# EUROPÄISCHE PATENTSCHRIFT

(11) **EP 3 338 093 B1**
(45) Veröffentlichungstag und Bekanntmachung des Hinweises auf die Patenterteilung: **29.04.2020**
(21) Anmeldenummer: 16753650.7
(22) Anmeldetag: 17.08.2016
(51) Int. Cl.: G01N 33/537

(54) **BAKTERIEN-SCHNELLTEST**
BACTERIA FAST TEST
TEST RAPIDE DE BACTERIES

(30) Priorität: 17.08.2015 EP 15181240
(43) Veröffentlichungstag der Anmeldung: 27.06.2018
(73) Patentinhaber: Swiss Analyze Bacteria AG, 8274 Tägerwilen (CH)
(72) Erfinder: EBERT, Dieter, 8274 Gottlieben (CH); REICHL, Mathias, 93309 Kehlheim (DE); LUBITZ, Werner, 3420 Kritzendorf (AT)
(74) Vertreter: Weickmann & Weickmann PartmbB
(86) Internationale Anmeldenummer: PCT/EP2016/069526
(87) Internationale Veröffentlichungsnummer: WO 2017/029332

(56) Entgegenhaltungen:
- EP-A1- 0 496 345
- EP-A1- 1 277 505
- "Mikrobiologischer Trinkwassertest in weniger als einer Stunde", , 1. April 2009 (2009-04-01), XP055228170, Gefunden im Internet: URL:http://www.wiley-vch.de/berlin/journal s/op/09-01/OP0901_S28-S31.pdf [gefunden am 2015-11-12]
- ULRICH REIDT ET AL: "REPRODUCIBLE FILTRATION OF BACTERIA WITH MICROMECHANICAL FILTERS", JOURNAL OF RAPID METHODS AND AUTOMATION IN MICROBIOLOGY, Bd. 16, Nr. 4, 1. Dezember 2008 (2008-12-01), Seiten 337-350, XP055169725, ISSN: 1060-3999, DOI: 10.1111/j.1745-4581.2008.00140.x
- ROMPRE A ET AL: "Detection and enumeration of coliforms in drinking water: Current methods and emerging approaches", JOURNAL OF MICROBIOLOGICAL METHODS, ELSEVIER, AMSTERDAM, NL, Bd. 49, Nr. 1, 1. März 2002 (2002-03-01), Seiten 31-54, XP002359233, ISSN: 0167-7012, DOI: 10.1016/S0167-7012(01)00351-7

## Beschreibung

Die Erfindung betrifft Verfahren und Vorrichtungen zur quantitativen Bestimmung von Bakterien.

Legionellen sind eine Gattung gramnegativer Bakterien. Optimale Bedingungen für ihre Vermehrung finden sie in Wasser in einem Temperaturbereich von 25-50 °C. Sie können in Anlagen für die Warmwassererzeugung und -verteilung, in Schwimmbädern, Luftwäschern für Klimaanlagen, Kühltürmen, Wassertanks und Wasserleitungen mit langen Stillstandszeiten vorkommen. Das Einatmen von legionellenhaltigem Wasser als Aerosol, z.B. beim Duschen, bei Klimaanlagen, durch Rasensprenger oder in Whirlpools, kann beim Menschen zu einer Infektion führen, die eine gefährliche, in vielen Fällen tödlich verlaufende Krankheit, die Legionellose, hervorrufen kann.

Die deutsche Trinkwasserverordnung schreibt daher eine regelmäßige Untersuchungspflicht auf Legionellen vor, die Inhaber von Trinkwasserinstallationen mit Großanlagen zur Trinkwassererwärmung betrifft, sofern aus diesen Wasser abgegeben wird und es dabei zu dessen Vernebelung, z.B. in Duschen, kommt. Diese Vorschrift gilt unter anderem für Krankenhäuser, Schulen, Kindergärten, Hotels und Pflegeheime. Diese Einrichtungen sind verpflichtet, einmal jährlich eine Untersuchung auf Legionellen durchzuführen. Seit neuerem müssen auch Eigentümer bzw. Vermieter von Mehrfamilienhäusern, Wohnungsbaugesellschaften und Hausverwaltungen entsprechende Untersuchungen in einem Intervall von jeweils drei Jahren durchführen.

In der Trinkwasserverordnung ist für Legionellen ein technischer Maßnahmenwert von 100 koloniebildenden Einheiten (KBE) je 100 ml festgelegt. Wird bei einer Untersuchung eine Überschreitung dieses Wertes festgestellt, muss unmittelbar eine Meldung an das zuständige Gesundheitsamt erfolgen.

Die Analytik im Rahmen der Untersuchungspflicht erfolgt mittels klassischer mikrobiologischer Nachweisverfahren. Die zu untersuchenden Trinkwasserproben werden im Labor aufgeteilt und in zwei parallelen Ansätzen gemäß ISO 11731 (1998) und DIN EN ISO 11731-2 (2008) untersucht. Ein Teil der Ausgangsprobe wird in einem Direktansatz und ein anderer Teil nach Filtration in Petrischalen mit Agar-Medium aufgebracht. Die Agar-Platten aus beiden Ansätzen werden zehn Tage bei einer konstanten Temperatur von 36 ± 2 °C im Brutschrank inkubiert. Danach werden während der Bebrütung gewachsene Kolonien von Legionellen gezählt und ausgewertet. Das quantitative Ergebnis wird in *"koloniebildenden Einheiten"* (KBE) bezogen auf 100 ml Probe angegeben.

Zur Bestätigung charakteristischer Legionellen-Kolonien werden mindestens fünf Kolonien parallel sowohl auf Cystein-haltigem Medium als auch auf einem Cystein-freien Medium mindestens zwei Tage lang subkultiviert. Da die Aminosäure Cystein für Legionellen essentiell ist, gilt der Nachweis als bestätigt, wenn die Kolonie auf dem Cystein-haltigen Medium wächst, nicht jedoch auf dem Cystein-freien Medium.

Ein Nachteil der oben genannten Analytik besteht darin, dass das mikrobiologische Nachweisverfahren kostspielig und zeitaufwändig ist. Ein weiterer Nachteil des Verfahrens besteht darin, dass die damit erhaltenen Ergebnisse eine Ungenauigkeit von zwischen ca. 15 und 35 % aufweisen.

EP 1 277 505 offenbart eine Vorrichtung, ein Verfahren und ein Durchflussanalysesystem zum Erfassen von immunogenen Partikeln wie etwa Bakterien mit einer Filtrationsmesszelle, die ein Filterteil zum Zurückhalten der Partikel und ein Sensorelement zum Empfangen eines spezifisch durch im Filtermaterial befindliche Partikel erzeugten Signals enthält. Die Partikel können mit einem spezifischen Enzym-markierten Antikörper direkt im Probenfluid markiert werden, bevor sie auf dem Filter abgelagert werden.

EP-A-0496345 offenbart ein Verfahren zum Nachweis und zur quantitativen Bestimmung von kariogenen Streptokokken, indem die Bakterien mit einem Antikörper markiert werden, der an die Bakterien gebundene Antikörper von freiem Antikörper durch Filtration durch ein Membranfilter abgetrennt und der gebundene Antikörper auf dem Filter nachgewiesen wird. Der Nachweis der gebundenen Antikörper erfolgt durch Enzymreaktion.

Friedberger und Homann offenbaren in *"Mikrobiologischer Trinkwassertest in weniger als einer Stunde"* (Optik & Photonik, 1. April 2009) einen Trinkwassertest, wobei eine Anreicherung von Bakterien auf einem Filter erfolgt. Die Bakterien können dabei durch fluoreszente Antikörper markiert werden.

Reidt et al. beschreiben in "Reproducible filtration of bacteria with micromechanical filters" (Journal of Rapid Methods and Automation in Microbiology, Bd. 16, Nr. 4, 1. Dezember 2008m S.337-350) einen Nachweis von Bakterien in Trinkwasser, bei dem die Bakterien auf einem Filter angereichert werden. Hierzu werden die auf dem Filter angelagerten Bakterien mit fluoreszenten Antikörpern markiert und durch Fluoreszenzmikroskopie nachgewiesen.

Rompre et al. beschreiben in "Detection and enumeration of coliforms in drinking water: current methods and emerging approaches" (Journal of Microbiological Methods, Elsener, Bd. 49, Nr. 1, 1. März 2002, S. 31-54) den Nachweis von Bakterien durch Anlagerung auf Membranfiltern und Nachweis durch markierte Antikörper, wobei beispielsweise Antikörper verwendet werden, die direkt mit einem Fluoreszenzfarbstoff gekoppelt sind.

Keines der oben genannten Verfahren offenbart die Verwendung von markierten Bakteriophagen als Markierungsreagenz zur quantitativen Bestimmung von Bakterien in einer Probe.

Die der vorliegenden Erfindung zugrunde liegende Aufgabe bestand somit darin, ein verbessertes Verfahren zur quantitativen Bestimmung von Legionellen und auch anderen Bakterien bereitzustellen, bei dem die Nachteile des Standes der Technik zumindest teilweise vermieden werden können. Insbesondere soll das Verfahren eine erheblich schnellere

Bestimmung mit hoher Genauigkeit erlauben.

Zur Lösung dieser Aufgabe werden neue Verfahren und Vorrichtungen zum quantitativen Nachweis von Bakterien bereitgestellt. Die Erfindung beruht auf einer Messung der markierten Bakterien, wobei das erhaltene Messsignal mit der Gesamtzahl der in der Probe vorhandenen Bakterien korreliert wird. Der Nachweis der Bakterien kann beispielsweise durch Lumineszenz-, Fluoreszenz- oder Raman-Strahlung erfolgen.

Ein erster Aspekt der Erfindung betrifft ein Verfahren zur quantitativen Bestimmung von Bakterien in einer Probe, umfassend die Schritte:
(a) Inkubieren der zu untersuchenden Probe mit einem an die zu bestimmenden Bakterien bindefähigen Markierungsreagenz, wobei das Markierungsreagenz ein markierter Bakteriophage, vorzugsweise ein fluoreszenz-, lumineszenz- oder Raman-markierter Bakteriophage ist, und in einem Überschuss bezüglich der nachzuweisenden Bakteriophagen verwendet wird,
(b) Entfernen von nicht an die Bakterien gebundenem Markierungsreagenz von den Bakterien durch eine für die zu bestimmenden Bakterien undurchlässige und für das Markierungsreagenz durchlässige Membran, wobei die zu bestimmenden Bakterien auf der Membran gesammelt werden,
(c) Bestimmen des an die gesammelten Bakterien gebundenen Markierungsreagenz vorzugsweise durch Lumineszenz-, Fluoreszenz- und/oder Ramanmessung und
(d) quantitatives Auswerten des Messsignals aus Schritt (c).

Das erfindungsgemäße Verfahren erlaubt eine schnelle und quantitative Bestimmung von Bakterien in einer Probe. Es kann direkt nach Probennahme und ohne vorherige Kultivierung der zu bestimmenden Bakterien durchgeführt werden. Die Inkubationszeit, die für die Bindung des Markierungsreagenz an die Bakterien erforderlich ist, liegt vorzugsweise bei einer Sekunde bis maximal fünf Minuten, besonders bevorzugt bis zu maximal 2,5 Minuten. Dies hat zur Folge, dass eine Gesamtmessdauer von weniger als zehn Minuten, vorzugsweise weniger als fünf Minuten erreichbar ist.

Vorzugsweise wird das Verfahren zur quantitativen Bestimmung von Legionellen verwendet. Es können jedoch auch andere Bakterien, insbesondere humanpathogene Bakterien wie Salmonellen, Listerien, coliforme Bakterien wie E. coli, z.B. EHEC, oder Krankenhauskeime wie MRSA bestimmt werden. Die Probe, in der die Bakterien bestimmt werden, kann eine Wisch- oder Wasserprobe, aber auch eine biologische Probe, z.B. eine Lebensmittelprobe aus Fleisch-, Milch- oder Eiprodukten, oder eine Körperflüssigkeit wie etwa Blut, Plasma, Serum, Urin etc. sein.

Die Bestimmung der Bakterien durch das erfindungsgemäße Verfahren erfolgt durch Verwendung eines mit den zu bestimmenden Bakterien bindefähigen Markierungsreagenz und Messung eines Signals, das von dem an die Bakterien gebundenen Markierungsreagenz stammt. Die Bestimmung kann beispielsweise durch Lumineszenzmessung, Fluoreszenzmessung und/oder durch Messung von Raman-Strahlung, z.B. mittels oberflächenverstärkter Raman-Spektroskopie (SERS) erfolgen. Dabei wird ein an die zu bestimmenden Bakterien bindefähiges Markierungsreagenz, vorzugsweise ein fluoreszentes, lumineszentes oder Raman-aktives Markierungsreagenz verwendet, das für die zu bestimmenden Bakterien selektiv ist, d.h. eine Struktur auf oder in den zu bestimmenden Bakterien erkennt, die nicht in anderen Bestandteilen der Probe vorkommt. Beispiele für das Markierungsreagenz sind markierte Nukleinsäuresonden, Antikörper, Bakteriophage und Kombinationen davon. Erfindungsgemäß ist das Markierungsreagenz ein markierter Bakteriophage.

In einer Ausführungsform kann ein fluoreszentes oder lumineszentes Markierungsreagenz verwendet werden, das Fluoreszenz- oder Lumineszenzstrahlung z.B. im Bereich von 200 nm bis 10 µm, insbesondere von 200 nm bis 2 µm emittiert. Besonders bevorzugte Emissionswellenlängen liegen im Bereich von 1300-1500 nm.

Fluoreszenz- oder lumineszenzmarkierte Nukleinsäuresonden, die zur Hybridisierung mit komplementären bakteriellen Nukleinsäuresequenzen verwendet werden, sind grundsätzlich bekannt und werden z.B. für die FISH-Technik verwendet. Die Nukleinsäuresonden sind üblicherweise Sonden auf Basis von Desoxyribonukleotid- und/oder Nukleinsäure-Analoga, z.B. LNA-Bausteinen. Sie können beispielsweise gegen ribosomale RNA- und/oder DNA-Sequenzen gerichtet sein, die für das nachzuweisende Bakterium spezifisch sind.

Auch markierte Antikörper, z.B. fluoreszenz- oder lumineszenzmarkierte Antikörper, d.h. polyklonale oder monoklonale Antikörper sowie Antikörperfragmente oder -derivate, die gegen ein für das nachzuweisende Bakterium spezifisches Antigen, z.B. ein auf der Bakterienoberfläche befindliches Antigen, gerichtet sind, sind bekannt.

Erfindungsgemäß ist das Markierungsreagenz ein Bakteriophage, der eine Markierung, z.B. eine Fluoreszenz- oder Lumineszenzmarkierung trägt und spezifisch Oberflächenstrukturen auf dem zu bestimmenden Bakterium erkennt. Bakteriophagen für Legionellen sind z.B. bei Lammetyn (Microb. Ecol. 56 (2000), 191-197) beschrieben. Bakteriophagen für Salmonella sind z.B. bei Atterbury et al. (Appl. Environ. Microbiol. 73 (2007), 4543-4549) Wichard et al. (J. Food Prot 2 (2003), 178-340) oder deLappe et al. (J. Med. Microbiol. 58 (2009) beschrieben. Bakteriophagen von Listeria sind z.B. bei Klumpp & Loessner (www. Ncbi. Nlm.nih.gov>PMC3827098) beschrieben. Bakteriophagen für MRSA sind z.B. bei Sahin et al. (Mikrobiol. Bul. 47 (2013), 27-34) beschrieben. Bakteriophagen für E. coli, z.B. T-Phagen, Lambda-Phagen oder andere, sind bei Sambrook et al. (Molecular Cloning, A Laboratory Manual) beschrieben.

Für das erfindungsgemäße Verfahren werden markierte Bakteriophagen, z.B. fluoreszenz- oder lumineszenzmarkierte Bakteriophagen in einem Überschuss bezüglich der nachzuweisenden Bakterien verwendet. Sie können etwa 10⁷-10⁹ Phagen pro Ansatz verwendet werden. Üblicherweise findet man, dass unter diesen Bedingungen jede Bakterienzelle mit etwa 50-400 Phagen, insbesondere mit etwa 50-250 oder 50-150 Phagen belegt wird. Eine E. coli Bakterienzelle kann beispielsweise mit etwa 70 Phagen belegt werden.

In einer weiteren bevorzugten Ausführungsform kann ein durch Raman-Spektroskopie nachweisbares Markierungsreagenz verwendet werden, z.B. ein Markierungsreagenz, das ein oberflächenverstärktes ("surface enhanced") Raman-spektroskopisch aktives Partikel enthält. Dieses Markierungsreagenz umfasst vorzugsweise ein metallisches Partikel, z.B. ein Gold- oder Silberpartikel und/oder ein Raman-Reportermolekül, das an eine an die nachzuweisenden Bakterien bindefähige Sonde gebunden ist. Das Raman-Reportermolekül kann z.B. aus einem Isothiocyanat- oder einem Multi-Schwefel-Fluoreszenzfarbstoff ausgewählt werden. Die Sonde ist ausgewählt aus Bakteriophagen, welche selektiv an die zu bestimmenden Bakterien binden.

Die Fluoreszenz- oder Lumineszenzgruppen des Markierungsreagenz können aus beliebigen bekannten organischen oder anorganischen Fluoreszenz- oder Lumineszenzfarbstoffen ausgewählt werden, die an das Markierungsreagenz vorzugsweise kovalent gebunden sind. Geeignete Fluoreszenzgruppen sind Fluoreszenzfarbstoffe, beispielsweise Fluoresceine, Rhodamine, Oxazine oder Phycoerythrine, Fluoreszenzproteine wie GFP oder Varianten davon sowie fluoreszierende Quantendots. Geeignete Nachweisgruppen zur oberflächenverstärkten Raman-Spektroskopie (SERS) sind Substanzen, die eine oder mehrere Isothiocyanat-Gruppen und/oder Schwefelatome umfassen.

Das Inkubieren der nachzuweisenden Bakterien mit dem Markierungsreagenz erfolgt in einer Reaktionskammer. Hierzu wird die zu untersuchende Probe in die Reaktionskammer eingebracht. Das Markierungsreagenz kann der Probe vor oder nach dem Einbringen in die erste Kammer in Kontakt gebracht werden. Die erste Kammer enthält ein Depot mit einer vorbestimmten Menge des Markierungsreagenz, das dort in flüssiger oder in trockener Form vorliegen kann. Vorzugsweise liegt das Markierungsreagenz in trockener und nach Kontakt mit der Probenflüssigkeit rekonstituierbarer Form vor. Die erste Kammer, in der die Inkubation der zu bestimmenden Bakterien mit dem Markierungsreagenz erfolgt, ist auf mindestens einer Seite durch eine Membran begrenzt sein. Alternativ kann die mit dem Markierungsreagenz versetzte Probe aus der Inkubationskammer in eine weitere Kammer geleitet werden, die mit einer Membran begrenzt ist. Diese Membran ist für die zu bestimmenden Bakterien undurchlässig, aber für das Markierungsreagenz, d.h. für ein nicht an Bakterien gebundenes Markierungsreagenz, durchlässig. Diese Membran weist beispielsweise einen Porendurchmesser von etwa 0,5-3 µm, z.B. 1-2 µm, auf. Geeignete Membranen, z.B. aus Kunststoffen wie Nylon, sind bekannt.

In einer bevorzugten Ausführungsform wird im erfindungsgemäßen Bestimmungsverfahren eine Messzelle verwendet, die mindestens zwei Kammern und eine zwischen den Kammern angeordnete Membran enthält, welche für die zu bestimmenden Bakterien undurchlässig und für nicht an die zu bestimmenden Bakterien gebundenes Markierungsreagenz durchlässig ist. Beispielsweise kann die Messzelle eine erste Kammer für die Inkubation und eine zweite Kammer zur Aufnahme abgetrennter Probenbestandteile und überschüssiger Markierung umfassen, die über eine Membran wie oben beschrieben in Verbindung stehen.

Nach Beendigung der Inkubation wird nicht an die Bakterien gebundenes Markierungsreagenz durch die Membran entfernt und die zu bestimmenden Bakterien auf der Membran gesammelt. Dieser Schritt kann durch Anlegen von Unterdruck an die Membran durchgeführt werden, so dass mit der Membran in Kontakt befindliche Probenflüssigkeit und nicht gebundenes Markierungsreagenz durch die Membran hindurch treten und die Bakterien auf der Membran gesammelt werden. Alternativ kann die Entfernung von Probenflüssigkeit und nicht gebundenem Markierungsreagenz auch durch Zentrifugation erfolgen. Vorzugsweise wird die von den Bakterien abgetrennte Probenflüssigkeit und das nicht gebundene Markierungsreagenz in eine hinter der Membran befindliche zweite Kammer geleitet.

Vor oder/und während der Inkubation kann eine homogene Durchmischung des Markierungsreagenz und der zu untersuchenden Probe erfolgen. Hierzu kann ein Vibrationselement vorgesehen werden, welches gegebenenfalls in der Messvorrichtung integriert ist. Nach der Inkubation können die markierten Bakterien gegebenenfalls ein- oder mehrmals gewaschen werden.

Die zur Inkubation vorgesehene erste Kammer ist so ausgestaltet, dass sie ein Probenvolumen aufnehmen kann, in dem sich eine für eine quantitative Bestimmung ausreichende Menge an Bakterien befindet. Üblicherweise ist die Reaktionskammer für ein Probenvolumen im Bereich von 0,5 ml oder größer, z.B. 1-100 ml, bevorzugt 5-50 ml, vorgesehen. Besonders bevorzugt ist ein Volumen im Bereich von etwa 10 ml.

Das erfindungsgemäße Verfahren beruht auf einer Bestimmung des an die gesammelten Bakterien gebundenen Markierungsreagenz durch Messung einer für die Markierungsgruppe charakteristischen Strahlung, z.B. von Fluoreszenz-, Lumineszenz- und/oder Raman-Strahlung. Hierzu wird Anregungslicht aus einer Lichtquelle auf die gesammelten Bakterien eingestrahlt und die von dem auf den Bakterien befindlichen Markierungsreagenz emittierte Fluoreszenz-, Lumineszenz- und/oder Raman-Strahlung gemessen. In einer bevorzugten Ausführungsform erfolgt die Messung, indem die gesammelten Bakterien nach Entfernen der Probenflüssigkeit und des überschüssigen Markierungsreagenz sowie gegebenenfalls einem oder mehreren Waschschritten direkt auf der Membran untersucht werden.

Als Lichtquelle wird vorzugsweise ein Laser, z.B. ein Quantenkaskadenlaser, verwendet. Dieser Laser steht in Verbindung mit einer geeigneten Optik, um eine Bestrahlung der gesammelten Bakterien, vorzugsweise eine flächige Bestrahlung der gesammelten Bakterien, vorzugsweise eine flächige Bestrahlung der auf der Membran gesammelten Bakterien, zu ermöglichen. Die Einkopplung des Lasersystems in die Reaktionskammer kann durch optisch transparente Fasern, z.B. Glasfasern, erfolgen.

Zum Nachweis von Fluoreszenz oder Lumineszenz kann als Laser eine Multimode- oder Singlemode-Laserdiode im UV-Bereich, z.B. mit einer Einstrahlwellenlänge von 350-420 nm, verwendet werden. Bevorzugt ist eine Einstrahlwellenlänge von 365 nm und 405 nm, die unter Verwendung von kommerziell verfügbaren Standardlaserdioden erzielt werden kann. Zum Nachweis von Raman-Strahlung kann auch ein Laser einer geeigneten Einstrahlwellenlänge im UV-, VIS- oder Nah-IR-Bereich verwendet werden.

Die Bestimmung des an die Bakterien gebundenen Markierungsreagenz kann durch Fluoreszenz-Spektroskopie, vorzugsweise durch Fluoreszenz-Korrelations-Spektroskopie und/oder durch Raman-Spektroskopie erfolgen. Dabei wird mittels einer geeigneten konfokalen Optik das von der Lichtquelle stammende Anregungslicht in die Probe fokussiert, um ein Anregungsvolumen zu erzeugen, in dem sich die zu bestimmenden Bakterien befinden.

Die von dem an die Bakterien gebundenen Markierungsreagenz emittierte Strahlung, z.B. Fluoreszenz-, Lumineszenz und/oder Raman-Strahlung, wird mit einem Photodetektor gemessen. Der Photodetektor kann z.B. eine Avalanche-Photodiode oder eine EMCCD Kamera sein. Vorzugsweise wird als Photodetektor ein Spektrometer eingesetzt, das eine spektrale Auflösung des emittierten Lichts über einen Bereich von mindestens 50 nm, mindestens 100 nm und bis zu 200 oder 250 nm ermöglicht. Vorzugsweise wird ein Photodetektor, z.B. ein Spektrometer mit einem Messbereich verwendet, der zwischen 200 bis 2000 nm liegt. Die optische Auflösung des Spektrometers beträgt 0,35-1 nm, vorzugsweise etwa 0,6-0,9 nm.

In einer Ausführungsform werden die markierten Bakterien durch oberflächenverstärkte Raman-Spektroskopie (SERS) bestimmt. Das Verfahren basiert auf der Anregung eines Raman-Reportermoleküls durch monochromatisches Laserlicht, wobei ein spezifisches Raman-Spektrum emittiert wird. Durch die Wechselwirkung des Raman-Reportermoleküls mit einem metallischen Nanopartikel, an das es gebunden ist, wird das Signal verstärkt. Bevorzugte metallische Nanopartikel bestehen aus einem Edelmetall wie Silber oder Gold und können unterschiedliche Strukturen und Größen aufweisen (Wang et al., Chem. Rev. 2013, 113, 1391-1428, EP 2 134 642 B1).

Das im Photodetektor erhaltene Messsignal wird quantitativ ausgewertet. Als Ergebnis erhält man die in der zu untersuchenden Probe befindliche Anzahl an Bakterien, die mit der gemessenen Fluoreszenz-, Lumineszenz- und/oder Raman-Strahlung korreliert. Die gemessene Anzahl an Bakterien entspricht einem bestimmten Wert koloniebildender Einheiten (KBE) pro Probenvolumen.

Die auf Basis des Messsignals bestimmte Anzahl an Bakterien kann anhand von Grenzwerten in eine oder mehrere Kategorien eingestuft werden. Diese Grenzwerte korrelieren mit einer für das jeweilige Verfahren spezifischen Fluoreszenzintensität. Beim Nachweis von Legionellen kann beispielsweise ein erster Grenzwert festgelegt werden, der einer Anzahl von 100 KBE pro 100 ml entspricht. Wenn die Anzahl der bestimmten Bakterien diesen Wert übersteigt, sind Maßnahmen zur Bekämpfung der Legionellen in dem entsprechenden Wassersystem zu veranlassen. Als weiterer Grenzwert kann eine Anzahl von 10.000 KBE pro 100 ml festgelegt werden. Wenn die Anzahl der bestimmten Bakterien diesen Wert übersteigt, sind Notfallmaßnahmen verbunden mit einer sofortigen Stilllegung des entsprechenden Wassersystems zu veranlassen.

Zur Unterstützung der quantitativen Auswertung kann eine Kalibrierung, z.B. durch die Verwendung von mindestens einem Referenzsignal, welches durch einen Referenzlaser erzeugt werden kann, durchgeführt werden. Alternativ kann eine Kalibrierung auch durch Verwendung interner Standards, z.B. fluoreszierender, lumineszierender oder Raman-aktiver Partikel, die eine von dem optisch nachweisbaren Markierungsreagenz unterschiedliche Fluoreszenz-, Lumineszenz- oder Ramanmarkierung tragen, erfolgen.

Ein weiterer Gegenstand der vorliegenden Erfindung ist eine Messzelle zur quantitativen Bestimmung von Bakterien, umfassend:
(i) eine erste Kammer, die ein Depot mit einem Markierungsreagenz für die zu bestimmenden Bakterien, enthält, wobei das Markierungsreagenz ein markierter Bakteriophage, vorzugsweise eine fluoreszenz-, lumineszenz- oder Raman-markierter Bakteriophage ist und wobei im Depot ein Überschuss markierter Bakteriophagen bezüglich der nachzuweisenden Bakterien in der ersten Kammer vorliegt,
(ii) eine zweite Kammer und
(iii) eine Membran, die zwischen der ersten Kammer und der zweiten Kammer angeordnet ist, wobei die Membran für die zu bestimmenden Bakterien undurchlässig und für nicht an die zu bestimmenden Bakterien gebundenes Markierungsreagenz durchlässig ist.

Noch ein weiterer Gegenstand der vorliegenden Erfindung ist eine Vorrichtung zur quantitativen Bestimmung von Bakterien, umfassend:
(i) eine Messzelle wie zuvor angegeben,
(ii) Mittel zum Abziehen von überschüssigem Markierungsreagenz aus der ersten Kammer in die zweite Kammer,
(iii) Mittel zur Anregung der Strahlung, z.B. der Fluoreszenz-, Lumineszenz- und/oder Raman-Strahlung, von markierten, auf der Membran gesammelten Bakterien und
(iv) Mittel zur quantitativen Auswertung eines von den gesammelten Bakterien stammenden Messsignals.

Die erfindungsgemäße Messzelle und Vorrichtung können in einem Verfahren zur quantitativen Bestimmung von Bakterien, insbesondere in dem zuvor beschriebenen erfindungsgemäßen Verfahren, verwendet werden. Besonders bevorzugt ist die Verwendung zur quantitativen Bestimmung von Legionellen in einer Wasserprobe.

Die Erfindung soll weiterhin durch die folgenden Abbildungen erläutert werden:

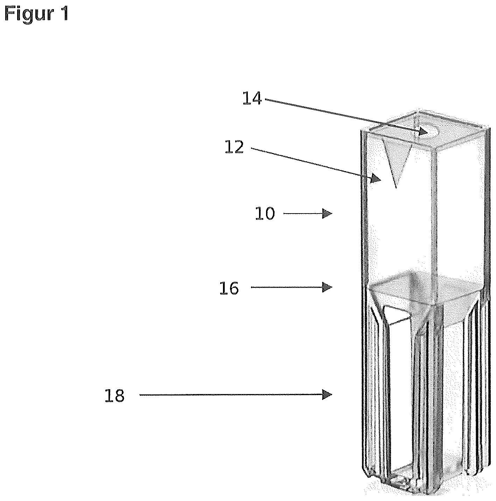

**Abbildung 1** zeigt eine Messzelle, die in Form einer Messküvette aufgebaut ist. Die Messzelle enthält eine Reaktionskammer oder erste Kammer (10) zur Aufnahme eines Probenvolumens von beispielsweise 10 ml. In der ersten Kammer (10) ist ein Markerdepot (12) mit einer vorbestimmten Menge an Markierungsreagenz, vorzugsweise in trockener Form, vorgesehen. Die erste Kammer (10) enthält auf einer Seite eine Öffnung (14) zur Aufnahme der Probe. Weiterhin steht die erste Kammer (10) über eine Membran (16) mit einer zweiten Kammer (18) in Verbindung. Die Membran (16) ist so ausgebildet, dass sie für in der ersten Kammer (10) befindliche Bakterien undurchlässig, aber für nicht an Bakterien gebundenes Markierungsreagenz durchlässig ist.
   Zur Messung wird die in der ersten Kammer (10) befindliche Flüssigkeit nach Inkubation mit dem Markierungsreagenz in die zweiten Kammer (18) geleitet. In der Probe vorhandene Bakterien mit daran gebundenem Markierungsreagenz werden auf der Membran (16) abgelagert. Nicht gebundenes Markierungsreagenz kann die Membran (16) durchdringen und gelangt mit der Flüssigkeit in die zweite Kammer (18). Die Messung der auf der Membran (16) abgelagerten Bakterien kann ohne weitere Maßnahme *in situ* erfolgen.

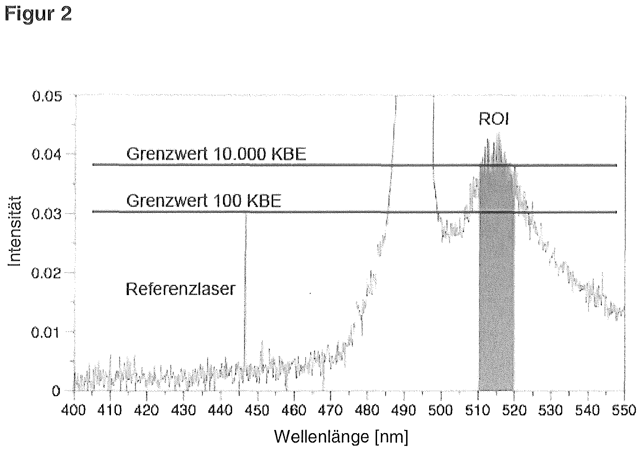

**Abbildung 2** zeigt das Ergebnis einer spektralen Messung an einer für die zu bestimmenden Bakterien, z.B. E. coli oder Legionellen, positiven Probe. Die Aufzeichnung des Messsignals erfolgt durch ein Spektrometer, dessen Messbereich vorzugsweise zwischen 350 und 550 nm liegt. Die optische Auflösung des Spektrometers liegt vorzugsweise bei 0,4-0,1 nm. Das von den zu bestimmenden Bakterien stammende Fluoreszenzsignal liegt im Bereich zwischen 510 und 520 nm (ROI). Durch einen Referenzlaser wird gewährleistet, dass die Amplitude des Spektrums einen korrekten Bezugswert hat. Die Auswertung umfasst die Festlegung von zwei Grenzwerten für Bakterienzahlen von 100 KBE pro 100 ml bzw. 10.000 KBE pro 100 ml. In der Abbildung übersteigt die Intensität des Messsignals den Grenzwert von 10.000 KBE. Folglich ist die bestimmte Probe extrem stark kontaminiert.

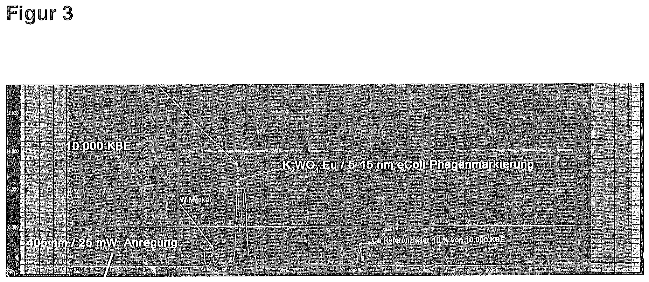

**Abbildung 3** zeigt das Ergebnis der Bestimmung einer Probe mit E. coli Bakterien. Als Markierungsreagenz wurden E. coli Phagen gekoppelt mit dem anorganischen Farbstoff K₂WO₄:Eu (Partikelgröße 5-15 nm) verwendet.

Die in der Probe enthaltenen Bakterien wurden nach Inkubation mit dem Markierungsreagenz in einer Reaktionskammer auf einer Membran abgelagert und dort bestimmt. Hierzu wurde die Membran mit einem Laser (405 nm/25 mW) homogen ausgeleuchtet und die bei einer Wellenlänge von 610-615 nm erzeugte Emissionsstrahlung quantitativ bestimmt. Die quantitative Umrechnung des in korrigierten Impulsen gemessenen Signals mit der Anzahl koloniebildender Einheiten (KBE) in der Probe war durch vorherige Eichung unter Verwendung von Standardproben mit einer bekannten Anzahl von E. coli Bakterien, z.B. im Bereich zwischen 10-10⁹ Zellen, möglich.

Im Spektrum ist außerdem der Peak eines Ca-Referenzlasers bei 702 nm zu erkennen, der mit einer Intensität entsprechend 10% des Grenzwerts von 10.000 KBE eingestrahlt wurde.

Im Bereich zwischen 590 und 600 nm ist noch ein - nicht zur quantitativen Bestimmung der Bakterien verwendetes - Signal des im Farbstoff enthaltenen Wolfram-Markers erkennbar.

Die Bestimmung der in Abbildung 3 gezeigten Probe ergab ein Signal, das einer Bakterienanzahl von 9.030 KBE entspricht. Durch Überprüfung mittels FACS konnte diese Bakterienanzahl verifiziert werden.

## Patentansprüche

1. Verfahren zur quantitativen Bestimmung von Bakterien in einer Probe umfassend die Schritte:
(a) Inkubieren der zu untersuchenden Probe mit einem an die zu bestimmenden Bakterien bindefähigen Markierungsreagenz, wobei das Markierungsreagenz ein markierter Bakteriophage, vorzugsweise ein fluoreszenz-, lumineszenz- oder Raman-markierter Bakteriophage ist, und in einem Überschuss bezüglich der nachzuweisenden Bakterien verwendet wird,
(b) Entfernen von nicht an die Bakterien gebundenem Markierungsreagenz von den Bakterien durch eine für die zu bestimmenden Bakterien undurchlässige und für das Markierungsreagenz durchlässige Membran, wobei die zu bestimmenden Bakterien auf der Membran gesammelt werden,
(c) Bestimmen des an die gesammelten Bakterien gebundenen Markierungsreagenz vorzugsweise durch Fluoreszenz-, Lumineszenz- und/oder Ramanmessung und
(d) quantitatives Auswerten des Messsignals aus Schritt (c).

2. Verfahren nach Anspruch 1,
**dadurch gekennzeichnet,**
**dass** die Bakterien ausgewählt werden aus Legionellen, Salmonellen, Listerien, coliformen Keimen wie E. coli, Krankenhauskeimen wie MRSA.

3. Verfahren nach Anspruch 1 oder 2,
**dadurch gekennzeichnet,**
**dass** zur Bestimmung eine Messzelle verwendet wird, umfassend
(i) eine erste Kammer, die gegebenenfalls ein Depot für das Markierungsreagenz enthält,
(ii) eine zweite Kammer und
(iii) eine Membran, die zwischen der ersten Kammer und der zweiten Kammer angeordnet ist, wobei die Membran für die zu bestimmenden Bakterien undurchlässig und für nicht an die zu bestimmenden Bakterien gebundenes Markierungsreagenz durchlässig ist, und
wobei nach Beendigung des Inkubationsschrittes nicht an die zu bestimmenden Bakterien gebundenes Markierungsreagenz aus der ersten Kammer entfernt und durch die Membran in die zweite Kammer geleitet wird.

4. Verfahren nach einem der Ansprüche 1 bis 3,
**dadurch gekennzeichnet,**
**dass** eine Membran mit einem Porendurchmesser von etwa 0,5-3 µm verwendet wird.

5. Verfahren nach einem der Ansprüche 1 bis 4,
**dadurch gekennzeichnet,**
**dass** vor und/oder während der Inkubation gemäß Schritt (a) eine Durchmischung des Markierungsreagenz und der zu untersuchenden Probe erfolgt.

6. Verfahren nach einem der Ansprüche 1 bis 5,
**dadurch gekennzeichnet,**
**dass** für die optische Bestimmung gemäß Schritt (c) ein Laser mit einer konfokalen Optik als Lichtquelle verwendet wird.

7. Verfahren nach Anspruch 6,
**dadurch gekennzeichnet,**
**dass** der Laser in einer integrierten Vorrichtung mit einem Spektrometer als Detektionseinheit verwendet wird.

8. Verfahren nach einem der Ansprüche 1 bis 7,
**dadurch gekennzeichnet,**
**dass** die quantitative Auswertung gemäß Schritt (d) die Einstufung der bestimmten Anzahl von Bakterien in eine oder mehrere Kategorien anhand von vorbestimmten Grenzwerten umfasst.

9. Verfahren nach einem der Ansprüche 1 bis 8,
**dadurch gekennzeichnet,**
**dass** für die quantitative Auswertung gemäß Schritt (d) mindestens ein Referenzsignal, insbesondere ein durch einen Laser erzeugtes Referenzsignal, verwendet wird.

10. Messzelle zur quantitativen Bestimmung von Bakterien nach einem Verfahren nach einem der Ansprüche 1-9, umfassend:
(i) eine erste Kammer, die ein Depot mit einer vorbestimmten Menge an Markierungsreagenz enthält, wobei das Markierungsreagenz ein markierter Bakteriophage, vorzugsweise ein fluoreszenz-, lumineszenz- oder Raman-markierter Bakteriophage ist und wobei im Depot ein Überschuss markierter Bakteriophagen bezüglich der nachzuweisenden Bakterien in der ersten Kammer vorliegt,
(ii) eine zweite Kammer und
(iii) eine Membran, die zwischen der ersten Kammer und der zweiten Kammer angeordnet ist, wobei die Membran für die zu bestimmenden Bakterien undurchlässig und für nicht an die zu bestimmenden Bakterien gebundenes Markierungsreagenz durchlässig ist.

11. Messzelle nach Anspruch 10,
**dadurch gekennzeichnet,**
**dass** das Volumen der ersten Kammer 2-20 ml beträgt.

12. Vorrichtung zur quantitativen Bestimmung von Bakterien, umfassend:
(i) eine Messzelle nach Anspruch 10 oder 11,
(ii) Mittel zum Abziehen von überschüssigem Markierungsreagenz aus der ersten Kammer in die zweite Kammer,
(iii) Mittel zur Anregung von Strahlung, vorzugsweise von Fluoreszenz-, Lumineszenz- und/oder Raman-Strahlung von markierten, auf der Membran gesammelten Bakterien und
(iv) Mittel zur quantitativen Auswertung eines von den gesammelten Bakterien stammenden Messsignals.

13. Verwendung der Messzelle nach Anspruch 10 oder 11 oder der Vorrichtung nach Anspruch 12 in einem Verfahren zur quantitativen Bestimmung von Bakterien in einem Verfahren nach einem der Ansprüche 1 bis 9.

14. Verwendung nach Anspruch 13 zur quantitativen Bestimmung von Legionellen in einer Wasserprobe.

## Claims

1. Method for quantitatively determining bacteria in a sample, the method comprising the steps of:
(a) incubating the sample to be examined together with a labelling reagent which is capable of binding to the bacteria to be determined, wherein the labelling reagent is a labelled bacteriophage, preferably a fluorescence-labelled, luminescence-labelled or Raman-labelled bacteriophage, and the labelling reagent is used in an excess with respect to the bacteria to be detected,
(b) removing labelling reagent which has not bound to the bacteria from the bacteria through a membrane which is impermeable to the bacteria to be determined and permeable to the labelling reagent, wherein the bacteria to be determined are collected on the membrane,
(c) determining the labelling reagent which has bound to the collected bacteria, preferably by fluorescence measurement, luminescence measurement and/or Raman measurement, and
(d) quantitatively evaluating the measurement signal from step (c).

2. Method according to claim 1,
**characterised in that**
the bacteria are selected from legionella, salmonella, listeria, coliform germs such as E. coli, and hospital germs such as MRSA.

3. Method according to either claim 1 or claim 2,
**characterised in that**
a measuring cell is used for the determination, comprising
(i) a first chamber which optionally contains a store for the labelling reagent,
(ii) a second chamber, and
(iii) a membrane which is arranged between the first chamber and the second chamber, the membrane being impermeable to the bacteria to be determined and permeable to labelling reagent which has not bound to the bacteria to be determined, and,
after the incubation step has ended, labelling reagent which has not bound to the bacteria to be determined being removed from the first chamber and passed through the membrane into the second chamber.

4. Method according to any of claims 1 to 3,
**characterised in that**
a membrane having a pore diameter of approximately 0.5-3 µm is used.

5. Method according to any of claims 1 to 4,
**characterised in that**
the labelling reagent and the sample to be examined are thoroughly mixed before and/or during the incubation according to step (a).

6. Method according to any of claims 1 to 5,
**characterised in that**
a laser having confocal optics is used as the light source for the optical determination according to step (c).

7. Method according to claim 6,
**characterised in that**
the laser is used in an integrated device having a spectrometer as a detection unit.

8. Method according to any of claims 1 to 7,
**characterised in that**
the quantitative evaluation according to step (d) comprises the classification of the determined number of bacteria into one or more categories based on predetermined limit values.

9. Method according to any of claims 1 to 8,
**characterised in that**
at least one reference signal, in particular a reference signal generated by a laser, is used for the quantitative evaluation according to step (d).

10. Measuring cell for quantitatively determining bacteria according to a method according to any of claims 1-9, the measuring cell comprising:
(i) a first chamber which contains a store having a predetermined amount of labelling reagent, wherein the labelling reagent is a labelled bacteriophage, preferably a fluorescence-labelled, luminescence-labelled or Raman-labelled bacteriophage, and wherein, in the store, there is an excess of labelled bacteriophages with respect to the bacteria to be detected in the first chamber,
(ii) a second chamber, and
(iii) a membrane which is arranged between the first chamber and the second chamber, wherein the membrane is impermeable to the bacteria to be determined and permeable to labelling reagent which has not bound to the bacteria to be determined.

11. Measuring cell according to claim 10,
**characterised in that**
the volume of the first chamber is 2-20 ml.

12. Device for quantitatively determining bacteria, comprising:
(i) a measuring cell according to either claim 10 or claim 11,
(ii) means for withdrawing excess labelling reagent from the first chamber into the second chamber,
(iii) means for exciting radiation, preferably fluorescence radiation, luminescence radiation and/or Raman radiation from labelled bacteria collected on the membrane, and
(iv) means for quantitatively evaluating a measurement signal originating from the collected bacteria.

13. Use of the measuring cell according to either claim 10 or claim 11 or the device according to claim 12 in a method for quantitatively determining bacteria in a method according to any of claims 1 to 9.

14. Use according to claim 13 for quantitatively determining legionella in a water sample.

## Revendications

1. Procédé pour la détermination quantitative des bactéries dans un échantillon comprenant les étapes :
a) incuber l'échantillon à analyser avec un réactif de marquage capable de se lier aux bactéries à déterminer, le réactif de marquage étant un bactériophage marqué, de préférence un bactériophage marqué par fluorescence, luminescence ou Raman, et étant utilisé en excès par rapport aux bactéries à détecter,
b) éliminer du réactif de marquage non lié aux bactéries à travers une membrane imperméable aux bactéries à déterminer et perméable au réactif de marquage, les bactéries à déterminer étant collectées sur la membrane,
c) déterminer le réactif de marquage lié aux bactéries collectées de préférence par mesure de fluorescence, de luminescence et/ou de Raman et
d) évaluer quantitativement le signal de mesure de l'étape c).

2. Procédé selon la revendication 1,
**caractérisée en ce que** les bactéries sont sélectionnées parmi les légionnelles, les salmonelles, les listeria, les germes conformes tels que E. coli, les germes hospitaliers tels que MRSA.

3. Procédé selon les revendications 1 ou 2,
**caractérisé en ce que**
l'on utilise pour la détermination une cellule de mesure comprenant
(i) une première chambre, qui contient éventuellement un dépôt pour le réactif de marquage,
(ii) une deuxième chambre et
(iii) une membrane disposée entre la première chambre et la deuxième chambre, dans laquelle la membrane est imperméable aux bactéries à déterminer et perméable au réactif de marquage non lié aux bactéries à déterminer, et
dans laquelle, après l'achèvement de l'étape d'incubation, le réactif de marquage non lié aux bactéries à déterminer est éliminé de la première chambre et passé à travers la membrane dans la deuxième chambre.

4. Procédé selon l'une quelconque des revendications 1 à 3,
**caractérisée en ce que**
l'on utilise une membrane ayant un diamètre de pores d'environ 0,5-3 µm.

5. Procédé selon l'une quelconque des revendications 1 à 4,
**caractérisé en ce**
**qu'**avant et/ou pendant l'incubation selon l'étape (a), on mélange le réactif de marquage et l'échantillon à examiner.

6. Procédé selon l'une des revendications 1 à 5,
**caractérisée en ce**
**qu'**un laser avec une optique confocale est utilisé comme source de lumière pour la détermination optique selon l'étape (c).

7. Procédé selon la revendication 6,
**caractérisée en ce que**
le laser est utilisé dans un dispositif intégré avec un spectromètre comme unité de détection.

8. Procédé selon l'une quelconque des revendications 1 à 7,
**caractérisée en ce que**
l'évaluation quantitative selon l'étape (d) comprend la classification du nombre déterminé de bactéries dans une ou plusieurs catégories sur la base de valeurs limites prédéterminées.

9. Procédé selon l'une des revendications 1 à 8,
**caractérisé en ce**
**qu'**au moins un signal de référence, en particulier un signal de référence généré par un laser, est utilisé pour l'évaluation quantitative selon l'étape (d).

10. Cellule de mesure pour la détermination quantitative de bactéries selon un procédé selon l'une des revendications 1 à 9, comprenant :
(i) une première chambre qui contient un dépôt avec une quantité prédéterminée de réactif de marquage, dans laquelle le réactif de marquage est un bactériophage marqué, de préférence un bactériophage marqué par fluorescence, luminescence ou Raman, et dans laquelle un excès de bactériophage marqué par rapport aux bactéries à détecter dans la première chambre est présent dans le dépôt,
(ii) une deuxième chambre, et
(iii) une membrane disposée entre la première chambre et la deuxième chambre, dans laquelle la membrane est imperméable aux bactéries à déterminer et perméable au réactif de marquage non lié aux bactéries à déterminer.

11. Cellule de mesure selon la revendication 10,
**caractérisée en ce que**
le volume de la première chambre est de 2-20 ml.

12. Dispositif pour la détermination quantitative de bactéries, comprenant :
(i) une cellule de mesure selon les revendications 10 ou 11,
(ii) des moyens pour retirer un excès de réactif de marquage de la première chambre dans la deuxième chambre,
(iii) des moyens pour exciter le rayonnement, de préférence la fluorescence, la luminescence et/ou le rayonnement Raman, des bactéries marquées collectées sur la membrane, et
(iv) des moyens pour évaluer quantitativement un signal de mesure provenant des bactéries collectées.

13. Utilisation de la cellule de mesure selon la revendication 10 ou 11 ou du dispositif selon la revendication 12 dans un procédé pour la détermination quantitative de bactéries dans un procédé selon l'une des revendications 1 à 9.

14. Utilisation selon la revendication 13 pour la détermination quantitative de légionnelles dans un échantillon d'eau.
